# EUROPÄISCHE PATENTSCHRIFT

(11) **EP 1 335 732 B1**
(45) Veröffentlichungstag und Bekanntmachung des Hinweises auf die Patenterteilung: **12.05.2004**
(21) Anmeldenummer: 02757717.0
(22) Anmeldetag: 12.03.2002
(51) Int. Cl.: A61K 31/66

(54) **NAHRUNGSMITTEL ZUR STEIGERUNG DER KOGNITIVEN LEISTUNGSFÄHIGKEIT**
FOOD ITEM FOR INCREASING COGNITIVE CAPACITY
ALIMENT DESTINE A AMELIORER LES CAPACITES COGNITIVES

(30) Priorität: 26.03.2001 AT 4822001
(43) Veröffentlichungstag der Anmeldung: 20.08.2003
(73) Patentinhaber: Giventis GmbH, 64546 Mörfelden-Walldorf (DE)
(72) Erfinder: GEISS, Kurt-Reiner, 63225 Langen/Hessen (DE)
(74) Vertreter: Richardt, Markus, Dipl.-Ing.
(86) Internationale Anmeldenummer: PCT/EP2002/002694
(87) Internationale Veröffentlichungsnummer: WO 2002/078464

(56) Entgegenhaltungen:
- EP-A- 0 711 559
- EP-A- 0 819 760
- EP-A- 0 946 156
- DE-A- 19 917 249
- US-A- 6 117 853

## Beschreibung

Die Erfindung betrifft ein Nahrungsmittel, insbesondere einen Riegel, zur Steigerung der kognitiven Leistungsfähigkeit.

Es ist allgemein bekannt, dass mit zunehmendem Alter ein Massenverlust des menschlichen Gehirns einhergeht, der bis zu ca. 100 g betragen kann. Dieser Massenverlust wird unter anderem bedingt durch eine Abnahme der Anzahl der Nervenzellen und der Dichte der synaptischen Verbindungen im neuronalen Netz.

Nach wissenschaftlichen Erkenntnissen steigt dabei der Cholesterinanteil im Gehirn an, während die Phospholipide abnehmen. Dieser langsame Degenerationsprozess geht mit einer Myelin-Zerstörung einher und bedingt konsekutiv die Abnahme des Phospholipidgehalts. Dadurch kommt es zu entsprechenden Störungen verschiedener physiologischer und biochemischer Zellfunktionen.

Es ist allgemein bekannt, dass sich diese Störungen im wesentlichen ungünstig auf die Hirnleistungsprozesse, voranging im kognitiven Bereich, auswirken. So nimmt z.B. das Gedächtnis bzw. die Merk- und Lernfähigkeit mit zunehmendem Alter kontinuierlich ab. Diese altersbedingte Hirnleistungsminderung wird gemäß der American Psychiatric Association und der American Psychological Association als Age Related Cognitive Decline (ARCD) und Age Associated Memory Impairment (AAMI) bezeichnet bzw. klassifiziert.

Obwohl die Eigenschaften einer stabilen Gedächtnisspur noch weitgehend unbekannt sind, geht man heute davon aus, dass das Gedächtnis als eine Art "biochemische Veränderung" im Neuronenschaltsystem gespeichert wird. Bei einem Merk- und Lernprozess wird eine gesteigerte Synthese neuer Ribonukleinsäuren (RNA) angenommen. Die RNA liefert dabei die Matrix für diese Proteinsynthese.

Kommt es nun im Rahmen des altersbedingten Degenerationsprozesses zu einer Abnahme des Phospholipidgehaltes, wird die Membranstruktur der Zellwände, die das Lösungsmedium für diese Proteine darstellen, zunehmend zerstört. Dadurch ist zum Beispiel der Aufbau einer neuen Gedächtnisspur (Kurzzeitgedächtnis) zunehmend erschwert.

Aus der EP 0 946 156 A ist eine Mischung aus Phosphatidylserin und Kohlenhydraten zur Herstellung eines ibuprofenhaltigen Getränks bekannt. Zur Herstellung einer biphasigen Lösung wird dabei phospholipidhaltiges Material mit dem Ibuprofenmedikament gemischt. Die Phospholipide haben dabei allein den technischen Zweck zur Herstellung einer Emulsion, so dass keine ölige I-buprofen-Schicht auf der Oberfläche des Getränks entsteht. Insbesondere wegen des Ibuprofengehalts ist dieses Getränk als Nahrungsmittelergänzung ungeeignet.

Der Erfindung liegt daher die Aufgabe zugrunde ein Nahrungsmittel, vorzugsweise einen Riegel zu schaffen, der die kognitive Leistungsfähigkeit, insbesondere bei Personen über dem 40. Lebensjahr, erhöht.

Die der Erfindung zugrunde liegende Aufgabe wird mit den Merkmalen der unabhängigen Patentansprüche jeweils gelöst. Bevorzugte Ausführungsformen sind in den abhängigen Patentansprüchen angegeben.

Ausgangspunkt der vorliegenden Erfindung ist die Erkenntnis, dass die Zufuhr von 100 mg bis 300 mg Phosphatidylserin pro Tag bei älteren Personen zu einer Verbesserung der kognitiven Leistungsfähigkeit, insbesondere der Merk- und Lernfähigkeit sowie einer Steigerung des Konzentrationsvermögens sowie der Aufmerksamkeit führen kann.

Phosphatidylserin ist ein Phospholipid und gehört als Nährstoff zur Gruppe der Lecithine. Durch verschiedene wissenschaftliche Untersuchungen ist belegt, dass Phosphatidylserin spezifische Wirkungen am Nervengewebe, insbesondere im Gehirn, zeitigt. Die maßgebliche Funktion von Phosphatidylserin im Nervengewebe bezieht sich auf die Bildung von Proteinen in der Zellmembranmatrix.

Diese Proteinstrukturen in der Zellmembran sind für sämtliche wichtige Schaltfunktionen an der Zelloberfläche verantwortlich. Dadurch wird die Signalübertragung bzw. Kommunikation zwischen den Hirnzellen gewährleistet und somit die Voraussetzung für eine optimale kognitive Leistungsfähigkeit geschaffen.

Für den Phospholipidmangel im Alter sind im Wesentlichen zwei unterschiedliche Ursachen relevant. Aus Crook TH, Adderly B (1998), "The memory cure", New York: Pocket Books, ist die Hypothese bekannt, dass der menschliche Körper von der Evolution her nicht auf ein Leben im hohen Alter programmiert ist. Die mit der normalen Ernährung aufgenommene Menge von Phosphatidylserin ist zwar ausreichend bis in das mittlere Lebensalter (etwa bis zum 45. Lebensjahr, welches einem vollen Lebensalter in prähistorischer Zeit entspricht), aber nicht mehr im späteren Lebensalter.

Eine weitere Ursache ist jedoch vor allem in den Änderungen der Ernährungsgewohnheiten zu sehen: Aufgrund einer fett- und cholesterinbewussten Ernährung wurde der Verzehr von Phosphatidylserin-haltigen (tierischen) Nahrungsmitteln deutlich eingeschränkt. Dies bedeutet, dass ca. 200 bis 400 mg pro Tag weniger Phosphatidylserin über die Lebensmittel zugeführt werden.

Entsprechend schlägt die Erfindung vor, den Phospholipidmangel im Alter durch eine Substitution bzw. Supplementierung über die Aufnahme von Phosphatidylserin in einem Nahrungsmittel zu beheben. Die Steigerung der kognitiven Leistungsfähigkeit bei Aufnahme von 100 mg bis 300 mg Phosphatidylserin pro Tag ist für Personen im Alter von mehr als 40 Jahren durch wissenschaftliche Studien belegt.

Dabei entfaltet Phosphatidylserin eine spezifische ernährungsphysiologische Wirkung. Bei diesen Dosierungen wird ein nutritiv und/oder situativ bedingter Mangel an Phosphatidylserin im Sinne einer gezielten Zufuhr ausgeglichen und in den Normbereich zurückgeführt. Durch den Zusatz von Phosphatidylserin zu einem Nahrungsmittel schafft die Erfindung daher ein sogenanntes "Functional Food", das über den reinen Ernährungszweck hinaus positive physiologische Wirkungen im Hinblick auf die kognitive Leistungsfähigkeit hat.

Vorzugsweise hat das erfindungsgemäße Nahrungsmittel einen relativ hohen Gehalt an Kohlenhydraten, wie z. B. Fructosesirup, Zucker und/oder Glucosesirup. In der spezifischen Verbindung der Aufnahme von Kohlenhydraten und Phosphatidylserin wird die Glucose-Aufnahme und somit der Glucosegehalt in den Hirnzellen deutlich erhöht. Dadurch ist kurzfristig eine besonders deutliche Steigerung der kognitiven Leistungsfähigkeit ermöglicht. Die Mindestmenge an Kohlenhydrate beträgt dabei vorzugsweise 15 g in Verbindung mit vorzugsweise 100 bis 300 mg Phosphatidylserin.

Vorzugsweise ist das erfindungsgemäße Nahrungsmittel als Riegel, vorzugsweise als Schokoriegel, ausgebildet. Als ernährungsphysiologische Wirksubstanz enthält der Riegel Phosphatidylserin, vorzugsweise aus Phosphatidylserin-haltigem Lecithin-Extrakt.

Ferner hat der Riegel vorzugsweise einen relativ hohen Kohlenhydratanteil, um die gewünschte Kombinationswirkung der kurzfristigen Verbesserung der kognitiven Leistungsfähigkeit bei der Aufnahme von Phosphatidylserin zu erreichen. Der Kohlenhydratanteil sollte dazu über 40 Gewichtsprozent, vorzugsweise uber 57 Gewichtsprozent liegen. Dies entspricht einem Anteil von uber 1 Gewichtsprozent, vorzugsweise 1,4 Gewichtsprozent Phosphatydilserin-haltigem Lecithin-Extrakt.

Ferner hat der Riegel vorzugsweise einen Anteil von mindestens 10 Gewichtsprozent, vorzugsweise 16 Gewichtsprozent Eiweiß und mindestens 15 Gewichtsprozent, vorzugsweise 27 Gewichtsprozent Fett. Ferner kann der Riegel mit Vitaminen angereichert werden. Der Überzug mit Schokolade, vorzugsweise Milchschokolade, erhöht die Genussfunktion.

Die Produktgröße des Riegels ist vorzugsweise mindestens 20 g, insbesondere 35 g. Der Riegel kann einzeln verpackt angeboten werden, was die Handhabung vereinfacht. Die Aufnahme von einem Riegel pro Tag ist für die nachhaltige Besserung der kognitiven Leistungsfähigkeit ausreichend; es können täglich jedoch auch mehrere Riegel verzehrt werden. Für die langfristige Steigerung der kognitiven Leistungsfähigkeit reichen aber bei regelmässigem Verzehr auch drei bis vier Riegel pro Woche noch aus.

Vorzugsweise hat der Riegel einen Wassergehalt von weniger als 3%, wodurch sich die Stabilität des Phosphatidylserin erhöht. Die Haltbarkeit kann dann über ein Jahr betragen, wobei die ernährungsphysiologische Wirkung von Phosphatidylserin voll erhalten bleibt.

Im Weiteren wird eine bevorzugte Ausführungsform der Erfindung mit Bezug auf die Zeichnung erläutert. Es zeigen;
- Fig. 1: die Molekularstruktur von Phosphatidylserin und
- Fig. 2: die Zusammensetzung einer bevorzugten Ausführungsform des erfindungsgemäßen Riegels.

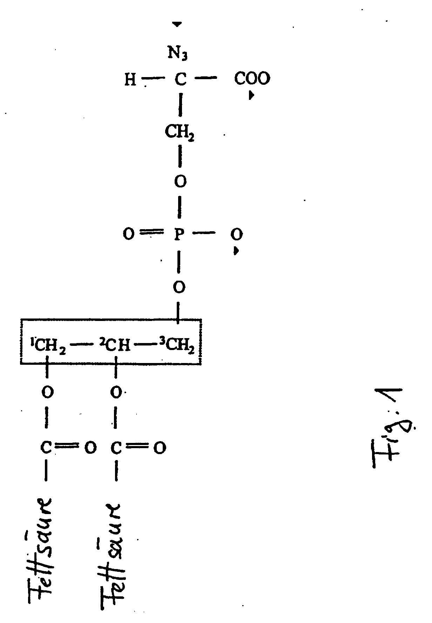

Die Fig. 1 zeigt die Molekularstruktur von Phosphatidylserin. Phosphatidylserin gehört zu der Gruppe der Phospholipide. Die Phospholipide werden unterteilt in die vier Gruppen Lecithine, Kephaline, Phosphatidylinosite und Sphingomyeline. Die Glyzerinphospholipide der Lecithin- und Kephalin-Gruppe zeigen einen identischen Aufbau: Die erste primäre und die sekundäre Hydroxylgruppe des Glyzerins ist mit einer Fettsäure, die zweite primäre Hydroxylgruppe ist mit Phosphat verestert.

Das Phosphat wiederum ist mit einer Hydroxiaminoverbindung verestert, hier sind die Stickstoffbasen Cholin, Ethanolamin und Serin zu finden. Phosphatidylserin ist den Kephalinen zugeordnet und ist daher auch unter dem Begriff "Serinkephalin" bekannt.

Phosphatidylserin besteht chemisch somit aus Fettsäuren, Glyzerin, Phosphat und Serin.

Neben der Herstellung von Phosphatidylserin aus Rinderhirn kommen die beiden folgenden bevorzugten Herstellungsverfahren zum Einsatz:
1. Aufkonzentrierung der in Soja-Lecithin natürlicherweise enthaltenen geringen Menge Phosphatidylserin von 0,2 bis 0,3% durch Extraktion und anschließende chromatographische Reinigung. Das Verfahren ist jedoch sehr aufwendig und auf eine Vielzahl organischer Lösemittel angewiesen.
2. Enzymatische Umwandlung der in handelsüblichem Lecithin enthaltenen Phospholipide, Phosphatidylcholin und Phosphatidylethanolamin in Phosphatidylserin, ohne Verwendung organischer Lösemittel. Bei diesem Verfahren handelt es sich um das Prinzip der enzymatischen Umesterung, wie es ähnlich bereits kommerziell für die Umesterung von Triglyzeride (z.B. für Kakaobutter-Ersatzstoffe oder mittelkettige Triglyzeride) durchgeführt wird. Die Umesterung von Fetten ist grundsätzlich ein lebensmitteltechnologisch übliches Verfahren zur Änderung der physikalischen Eigenschaften eines Fettes und findet eine sehr breite Anwendung z.B. mit folgenden Zielsetzungen: Herstellung von Fettkomponenten mit bestimmten Schmelzeigenschaften (Konsistenz) für Margarinekompositionen, Backfette und Backfettkomponenten sowie Süßwarenfette.

Vorzugsweise wird zur Herstellung von pflanzlichem Phosphatidylserin vorrangig die enzymatische Umwandlung eingesetzt.

Aus der DE 199 172 49 ist ein Verfahren zur Herstellung von Phosphatidylserin bekannt, bei welchem Lecithin in Wasser dispergiert. Phospholipase D und Kalziumchorid werden in Wasser gelöst in die Dispersion überführt. Nach 10 bis 20 Stunden Rühren bei Raumtemperatur wird das Calciumsalz des Phosphatidylserin aus der wässrigen Phase abgetrennt, das freie L-Serin und Cholin im Phosphatidylserin wird ausgewaschen. Durch abschließende Ethanolextraktion werden Phosphatidylserin bzw. Phosphatidylserin angereicherte Produkte erhalten, die keine Restenzymaktivität aufweisen.

Ferner ist aus der US 5,965,413 ein Verfahren zur Herstellung von Phosphatidylserin mit einer ungesättigten Fettsäure als Seitenkette bekannt. Die grundsätzliche Verwendungsmöglichkeit von Phosphatidylserin zur Steigerung der kognitiven Leistungsfähigkeit ist ferner aus US 5,900,409 und US 6,117,853 an sich bekannt.

Anhand von tierexperimentellen Untersuchungen können folgende Aussagen als gesichert betrachtet werden. Die Applikation von Phosphatidylserin
- beugt der Neuronenatrophie vor,
- verhindert teilweise den altersbedingten Abbau der Rezeptoren für den Nervenwachstumsfaktor,
- fördert die Bildung von Nervenwachstumsfaktoren (eine Phosphatidylserin-spezifische Wirkung, die bei anderen Phospholipiden nicht festgestellt werden konnte),
- normalisiert das Cholesterin-/Phospholipidverhältnis im alternden Gehirn,
- verbessert den ATPase abhängigen Ionentransport über die Zellmembran und
- normalisiert die Proteinkinase-C-Balance.

Darüber hinaus ist die Bioverfügbarkeit von oral appliziertem Phosphatidylserin als gut zu bewerten (nach oraler Gabe ist radioaktiv markiertes Phosphatidylserin nach 30 Minuten im Blut feststellbar und überquert anschließend nach Passage der Leber die Blut-Hirn-Schranke).

Durch neurophysiologische Testverfahren ist darüber hinaus die Steigerung der kognitiven Leistungsfähigkeit bei Probanden im Alter zwischen 40 und 80 Jahren mit sogenanntem Age Related Cognitive Decline (ARCD) und sogenanntem Age Associated Memory Impairment (AAMI) nachgewiesen.

Die nachfolgend aufgelisteten Tests stellen eine Auswahl der Untersuchungsverfahren dar, die zum Nachweis der Steigerung der kognitiven Leistungsfähigkeit eingesetzt werden:
a) Nachweis der Steigerung von Aufmerksamkeit und Konzentration:
   Diller L et al. (1974): Studies in Cognition and Rehabilitation in Hemiplegia (Letter Cancellation Test). Rehabilitation Monograph Nr. 50. Institute of Rehabilitation Medicine. New York: University Medical Center.
   Schmith A (1973): Symbol Digit Modalities Test. Los Angeles: Western Phosphatidylserinychological Services
   Wechsler D et al. (1955): Adult Intelligence Scale Manual (Digit-Symbol und Digit Span (Forward/Backward)). New York: Phosphatidylserinychological Corporation
   Wechsler D (1970): Echelle d'intelligence des Wechsler pour adultes: WAIS: 2èEdition. Paris, centre de Phosphatidylserinychologie Appliquée.
b) Zum Nachweis der Steigerung der Merk- und Lernfähigkeit
   Rey 15-Word Test for short and long-term verbal memory Rey A (1964): Léxamen clinique en Phosphatidylserinychologie (Rey 15-Word Test for short and long-term verbal memory). Paris: Presses Universitaires de France
   Block Tapping Test (BTT)
      Milner B (1971): Interhemisphenic differences in the localisation of Phosphatidylserinychological processes in men (Block Tapping Test (BTT)). British medical Bulletin, 27: 272
   SET Test
      Isaacs B et al. (1972): The Set Test, a rapid Test of Mental Function in Old People. Age and Agening, 1:222
   The five words from the Randt-Memory Test
      Randt CT et al. (1980): A memory test for longitudinal measurement of mild to moderate deficites (The five words from Randt-Memory Test). Clinical NeuroPhosphatidylserinychology, 2:184

Darüber hinaus wurde in der Mehrzahl der vorliegenden Studien der Verhaltenstest nach der Plutchik Geriatric Rating Scale vorgenommen. (Plutchik R et al. (1970): Reliability and validity of a scale for assessing the functioning of geriatric patients (Plutchik Geriatric Rating Scale). Journal of the American Geriatric Society, 18,6:491-500).

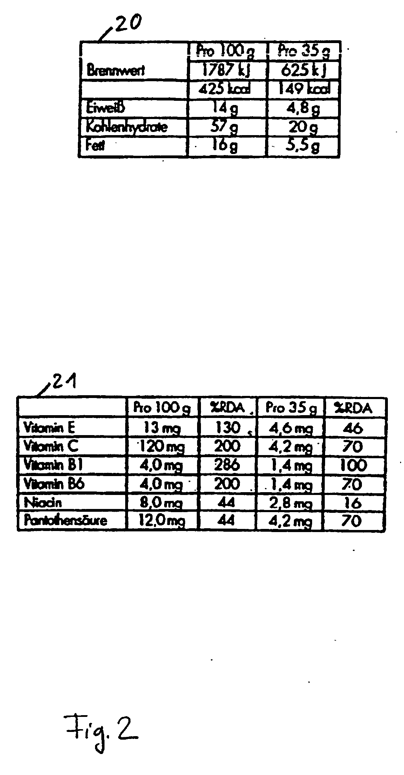

Die Fig. 2 zeigt die Tabellen 20 und 21 bezüglich einer Ausführungsform des erfindungsgemässen Schokoriegels. Die Tabelle 20 gibt den Brennwert des Schokoriegels pro 100 g bzw. pro 35 g sowie die jeweiligen Anteile von Eiweißen, Kohlenhydraten und Fetten an.

Die Tabelle 21 gibt die Zusammensetzung des Schokoriegels hinsichtlich der Vitamine E, C, B1, B6 sowie hinsichtlich Niacin und Pantothensäure an.

Die Zutaten pro 100 g des Schokoriegels sind: Fruktosesirup, Zucker, Magermilchpulver, Kakaobutter, Milchpulver, Milcheiweiß, Süßmolkepulver, Dextrose, pflanzliches Öl gehärtet, Kakaomasse, Maltodextrin, modifizierte Stärke, Reisextrudat, 1,4 g Lecithin-Extrakt, Kaffee-Extrakt, Aroma, Emulgator Lecithine, 120 mg Vitamin C, getrocknetes Ei-Albumin, 13,2 Pantothenat, 13 mg Vitamin E, 8 mg Niacin, 4 mg Vitamin B1, 4 mg Vitamin B6 sowie 200 mg Phosphatidylserin aus Lecithin-Extrakt. Die Produktgröße des Riegels ist vorzugsweise 35 g.

Durch den Verzehr von täglich einem oder mehrerer der Schokoriegel kommt es einerseits kurzfristig nach der Einnahme des Riegels zu einer Steigerung der kognitiven Leistungsfähigkeit und andererseits langfristig zu einer nachhaltigen Verbesserung der kognitiven Fähigkeiten, die zum Beispiel nach einem Zeitraum von ein bis drei Wochen eintritt. Dadurch lassen sich Verbesserungen sowohl hinsichtlich ARCD als auch hinsichtlich AAMI erreichen.

Die Erfindung ist jedoch keineswegs auf Riegel beschränkt; vielmehr kann die Zufuhr von Phosphatidylserin in einer Menge von täglich ca. 100 bis 300 mg auch über andere Nahrungs- bzw. Lebensmittel, insbesondere sogenannte "Functional Food"-Produkte, zum Beispiel Getränke, Brotaufstriche, Schokoladen- und Süßwarenerzeugnisse, Milch, Milchprodukte, diätische Lebensmittel, Getreideerzeugnisse, etc. erfolgen. Solche Nahrungsmittel sollten vorzugsweise einen relativ hohen Kohlenhydratanteil enthalten, um die gewünschte Kombinationswirkung mit Phosphatidylserin hinsichtlich der kurzfristigen Steigerung der kognitiven Leistungsfähigkeit durch Erhöhung des Glucosespiegels im Gehirn zu erreichen.

## Patentansprüche

1. Nahrungsmittel zur Steigerung der kognitiven Leistungsfähigkeit mit mindestens 100 mg Phosphatidylserin, vorzugsweise 200 mg bis 300 mg Phosphatidylserin, und mit mindestens 15 g Kohlenhydraten.

2. Nahrungsmittel nach Anspruch 1 zur Vermeidung und Vorbeugung von Aufmerksamkeits- und Konzentrationsschwierigkeiten, zur Vermeidung und Vorbeugung von Gedächtnisstörungen und Lernstörungen, zur Verbesserung der Aufmerksamkeit und Konzentration, zur Verbessung des Gedächtnis und der Lernfähigkeit und/oder zur Anwendung bei Lern- und Schulungsprozessen.

3. Nahrungsmittel nach Anspruch 1 oder 2, bei dem es sich um ein "Functional Food"-Produkt handelt, zum Beispiel Getränke, Brotaufstriche, Schokoladenerzeugnisse, Süßwarenerzeugnisse, Milch, Milchprodukte, diätische Lebensmittel und Getreideerzeugnisse.

4. Riegel zur Steigerung der kognitiven Leistungsfähigkeit mit mindestens 100 mg Phosphatidylserin, vorzugsweise 200 mg bis 300 mg Phosphatidylserin, und mit mindestens 15 g Kohlenhydraten.

5. Riegel nach Anspruch 4 mit einem Gewicht von mindestens 20 g, vorzugsweise 35 g.

6. Riegel zur Steigerung der kognitiven Leistungsfähigkeit mit mindestens 40 Gewichtsprozent Kohlenhydraten, vorzugsweise 57 Gewichtsprozent Kohlenhydraten, und mit mindestens 1 Gewichtsprozent, vorzugsweise 1,4 Gewichtsprozent Phosphatidylserin-haltigem Lecithin-Extrakt.

7. Riegel nach einem der Ansprüche 5 bis 6 mit mindestens 10 Gewichtsprozent Eiweiß, vorzugsweise 16 Gewichtsprozent Eiweiß.

8. Riegel nach einem der Ansprüche 5 bis 7, der mit Vitaminen angereichert ist und einen Schokoladenüberzug aufweist.

9. Riegel nach einem der Ansprüche 5 bis 8 mit mindestens 15 Gewichtsprozent Fett, vorzugsweise 27 Gewichtsprozent Fett.

10. Riegel nach einem der Ansprüche 5 bis 9 mit einem Wassergehalt von weniger als 3%.

11. Riegel nach einem der Ansprüche 5 bis 10 zur Vermeidung und Vorbeugung von Aufmerksamkeits- und Konzentrationsschwierigkeiten, zur Vermeidung und Vorbeugung von Gedächtnisstörungen und Lernstörungen, zur Verbesserung der Aufmerksamkeit und Konzentration, zur Verbessung des Gedächtnis und der Lernfähigkeit und/oder zur Anwendung bei Lern- und Schulungsprozessen.

12. Riegel nach einem der Ansprüche 5 bis 11, bei dem es sich um ein "Functional Food"-Produkt handelt.

## Claims

1. A food for increasing the cognitive functional capacity with a minimum of 100 mg of phosphatidyl serine, preferably 200 to 300 mg of phosphatidyl serine, and with a minimum of 15 g of carbohydrates.

2. The food of Claim 1 to prevent and protect against attention deficit disorders and lack of concentration, to prevent and protect against impaired memory and learning disorders, to improve attentiveness and concentration, to improve memory and learning ability and/or to use in learning and training processes.

3. The food of Claim 1 or 2 which is a "functional food" product, for example, beverages, bread spreads, chocolate products, candy products, milk, dairy products, diet foods, and cereals.

4. A bar for increasing the cognitive functional capacity with a minimum of 100 mg of phosphatidyl serine, preferably 200 mg to 300 mg of phosphatidyl serine, and with a minimum of 15 g of carbohydrates.

5. The bar of Claim 4 with a weight of at least 20 g, preferably 35 g.

6. A bar for increasing the cognitive functional capacity with a minimum of 40 wt% carbohydrates, preferably 57 wt% carbohydrates, and with a minimum of 1 wt%, preferably 1.4 wt% lecithin extract containing phosphatidyl serine.

7. The bar of Claim 5 or 6 with a minimum of 10 wt% protein, preferably 16 wt% protein.

8. The bar of any one of Claims 5-7 which is enriched with vitamins and which has a chocolate coating.

9. The bar of any one of Claims 5-8 with a minimum of 15 wt% fat, preferably 27 wt% fat.

10. The bar of any one of Claims 5-9 with a water content of less than 3%.

11. The bar of any one of Claims 5-10 to prevent and protect against attention deficit disorders and lack of concentration, to prevent and protect against impaired memory and learning disorders, to improve attentiveness and concentration, to improve memory and learning ability and/or to use in learning and training processes.

12. The bar of any one of Claims 5-11 which is a "functional food" product.

## Revendications

1. Aliment destiné à améliorer les capacités cognitives, contenant au moins 100 mg de phosphatidylsérine, de préférence 200 à 300 mg de phosphatidylsérine, et avec au moins 15g de glucides.

2. Aliment selon la revendication 1, destiné à éviter et à prévenir les difficultés d'attention et de concentration, à éviter et à prévenir les défaillances de mémoire et les difficultés à apprendre, à améliorer l'attention et la concentration, à améliorer la mémoire et la capacité à apprendre et/ou à utiliser dans les processus d'apprentissage et de formation.

3. Aliment selon la revendication 1 ou 2, dans lequel il s'agit d'un produit « aliment fonctionnel », par exemple des boissons, des produits à tartiner, des produits chocolatés, des produits sucrés, du lait, des produits laitiers, des aliments diététiques et des produits aux céréales.

4. Barre destinée à améliorer les capacités cognitives, contenant au moins 100 mg de phosphatidylsérine, de préférence 200 à 300 mg de phosphatidylsérine, et avec au moins 15g de glucides.

5. Barre selon la revendication 4, avec un poids d'au moins 20 g, de préférence 35 g.

6. Barre destinée à améliorer les capacités cognitives, contenant au moins 40 % en poids de glucides, de préférence 57 % en poids de glucides, et avec au moins 1 % en poids d'extrait de lécithine contenant de la phosphatidylsérine, de préférence 1,4 %.

7. Barre selon l'une quelconque des revendications 5 à 6, contenant au moins 10 % en poids de blanc d'oeuf, de préférence 16 % en poids de blanc d'oeuf.

8. Barre selon l'une quelconque des revendications 5 à 7, enrichie en vitamines et qui présente une couche de chocolat.

9. Barre selon l'une quelconque des revendications 5 à 8, ayant au moins 15 % en poids de graisse, de préférence 27 % en poids de graisse.

10. Barre selon l'une quelconque des revendications 5 à 9, ayant une teneur en eau inférieure à 3 %.

11. Barre selon l'une quelconque des revendications 5 à 10, destinée à éviter et à prévenir les difficultés d'attention et de concentration, à éviter et à prévenir les défaillances de mémoire et les difficultés à apprendre, à améliorer l'attention et la concentration, à améliorer la mémoire et la capacité à apprendre et/ou à utiliser dans les processus d'apprentissage et de formation.

12. Barre selon l'une quelconque des revendications 5 à 11, dans laquelle il s'agit d'un produit « aliment fonctionnel ».
